# EUROPÄISCHE PATENTSCHRIFT

(11) **EP 0 973 787 B1**
(45) Veröffentlichungstag und Bekanntmachung des Hinweises auf die Patenterteilung: **29.01.2003**
(21) Anmeldenummer: 98924089.0
(22) Anmeldetag: 30.03.1998
(51) Int. Cl.: C07H 15/04

(54) **VERFAHREN ZUR HERSTELLUNG VON GLUCOSESUSPENSIONEN MIT ANSCHLIESSENDER ENTWÄSSERUNG**
PROCESS FOR PREPARING GLUCOSE SUSPENSIONS AND SUBSEQUENT DEHYDRATION
PROCEDE DE PREPARATION DE SUSPENSIONS DE GLUCOSE AVEC DESHYDRATATION CONSECUTIVE

(30) Priorität: 07.04.1997 DE 19714255
(43) Veröffentlichungstag der Anmeldung: 26.01.2000
(73) Patentinhaber: Cognis Deutschland GmbH & Co. KG, 40589 Düsseldorf (DE)
(72) Erfinder: ESKUCHEN, Rainer, D-40764 Langenfeld (DE); HASSE, Eiko, D-40589 Düsseldorf (DE); GUTSCHE, Bernhard, D-40724 Hilden (DE)
(86) Internationale Anmeldenummer: EP9801851
(87) Internationale Veröffentlichungsnummer: WO98045307

(56) Entgegenhaltungen:
- WO-A-93/16088
- WO-A-93/18046
- DE-A- 4 321 838

## Beschreibung

Die Erfindung betrifft ein Verfahren zur Herstellung von Glucosesuspensionen mit anschliessen der Entwässerung.

### Stand der Technik

Alkylglucoside stellen wichtige nichtionische Tenside dar, die infolge ihres Verhaltens, das in vielen Fällen dem anionischer Tenside entspricht, und ihrer ausgezeichneten ökologischen und dermatologischen Eigenschaften Eingang in manuelle Spülmittel und kosmetische Zubereitungen finden. Zu ihrer Herstellung kann man von Glucose oder Glucosesirup ausgehen, die gegebenenfalls über die Zwischenstufe der Butylglucoside mit einem Überschuß an höheren Alkoholen sauer acetalisiert werden. Entsprechende technische Verfahren reichen bis in die Jahre 1968/70 zurück [vgl. **US 3,547,828, US 3,839,318** Rohm & Haas). Die Herstellung der Glucoside erfolgt üblicherweise ausgehend von festen Glycosen, wie beispielsweise Glucosemonohydrat, diskontinuierlich in einem Rührkessel mit anschließender Fettalkoholverdampfung. Es hat aber in der Vergangenheit nicht an Verfahren gemangelt, die für die Acetalisierung kontinuierliche Verfahren vorschlagen, beispielsweise unter Verwendung von Dünnschichtverdampfem **[EP-A1 0501032** (Hüls), **DE-A1 4231833** (Henkel)] oder Gegenstromreaktionskolonnen **[EP-A1 0482325, EP-A1 0514627** (Hüls)]. Diese Verfahren setzen den Einsatz von Glucosesirup zusammen mit Butanol voraus, da es andernfalls leicht zu Ankrustungen, Verklebungen und hohen Anteilen nicht umgesetzter Glucose kommen kann. Der Weg über die Bildung von Butylglucosiden als Zwischenstoffen, die nachfolgend mit Fettalkoholen weiter zu den Endprodukten umgesetzt werden müssen, ist jedoch technisch aufwendig und daher nicht erwünscht. Aus der internationalen Patentanmeldung WO 93/18046 ist ein Verfahren zur sauren Acetalisierung von Glucose mit Fettalkoholen bekannt, in dem zunächst wässrige Glucosesirupe und Fettalkohole in einem Turbinentrockner entwässert und anschließend acetalisiert werden.

Die Aufgabe der vorliegenden Erfindung hat darin bestanden, ein Verfahren zur direkten sauren Acetalisierung von Glucosesirup mit Fettalkoholen zu Verfügung zu stellen, das die Mitverwendung von Butanol nicht erfordert. Vorzugsweise sollte das Verfahren eine kontinuierliche Arbeitsweise ermöglichen.

### Beschreibung der Erfindung

Gegenstand der Erfindung ist ein Verfahren zur Herstellung von Glucosesuspensionen, bei dem man aufgeschmolzenen Glucosesirup bei 25 bis 45 °C in Fettalkohole einträgt, den als Feststoff ausfallenden Glucosesirup in den Fettalkohol dispergiert und anschließend die Glucose/Fettalkohol-Suspension entwässert.

Überraschenderweise wurde gefunden, daß sich auch wäßriger Glucosesirup problemlos mit Fettalkoholen sowohl diskontinuierlich als auch kontinuierlich acetalisieren läßt, ohne daß die Zwischenstufe der Butylglucoside durchlaufen werden muß, wenn man den aufgeschmolzenen Glucosesirup bei 25 bis 45° in Fettalkohole einträgt und den als Feststoff ausgefallenen Glucosesirup in dem Fettalkohol dispergiert.

### Glucosesirup

Im Rahmen der vorliegenden Erfindung sind unter Glucosesirup vorzugsweise raffinierte wäßrige Lösungen von D-Glucose, Maltose und höhere Polymere der Glucose, beispielsweise Oligosaccharide oder Dextrine zu verstehen, die im allgemeinen durch saure Hydrolyse und/oder durch enzymatischen Abbau von Stärke hergestellt werden. Besonders bevorzugt sind Glucosesirupe mit einem Feststoffanteil von 50 bis 85, insbesondere 75 bis 80 Gew.-% und einem DP1 Grad (monomerer Glucosegehalt) von 80 bis 99,9, insbesondere 94 bis 99,5 Gew.% bezogen auf den Feststoff.

### Suspensionen von Glucosesirup in Fettalkoholen

Zum Einsatz von Glucosesirup ist es erforderlich, diesen vor der Acetalisierung in eine Zubereitungsform zu bringen, die seine Handhabung erleichtert und insbesondere verhindert, daß es im Laufe der Reaktion zu Zersetzungsreaktionen kommt. Hierzu wird der Fettalkohol auf 25 bis 40°C vorgewärmt und in diesen der aufgeschmolzene Glucosesirup unter starkem Rühren oder unter Zuhilfenahmen eines Inline-Mischers dispergiert. Unter den genannten Bedingungen fällt der Glucosesirup als Feststoff aus und es bildet sich eine stabile Glucosesirup/Fettalkohol-Suspension, die problemlos in die direkte Acetalisierung eingesetzt werden kann. Ein weiterer Gegenstand der Erfindung betrifft daher die Verwendung von Suspensionen aus Glucosesirup und Fettalkoholen als Rohstoffe zur Herstellung von Alkyl- und/oder Alkenyloligoglykosiden.

### Fettalkohole

Unter Fettalkoholen sind primäre aliphatische Alkohole der Formel **(I)** zu verstehen,

**R**^{**1**}**OH (I)**

in der R¹ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 8 bis 18 Kohlenstoffatomen wie beispielsweise Kokos-, Palm-, Palmkern- oder Talgfettalkohol. Vorzugsweise werden die Glykosen und die Fettalkohole im molaren Verhältnis 1 : 1 bis 1 : 5, insbesondere 1 : 2 bis 1 : 3 eingesetzt. Mit diesen Einsatzverhältnissen werden Glykoside hergestellt, die einen DP im Bereich von 1,0 bis 1,8, vorzugsweise 1,3 bis 1,7 aufweisen.

### Katalysatoren

Die Wahl der sauren Katalysatoren ist an sich wenig kritisch, im Hinblick auf Produktfarbe, Schaumentwicklung bei der Acetalisierung, Ausbeute und gewünschtem durchschnittlichen Polymerisationsgrad (DP) hat sich der Einsatz von Alkylbenzolsulfonsäuren wie beispielsweise p-Toluolsulfonsäure oder Dodecylbenzolsulfonsäure als besonders vorteilhaft erwiesen. Die Katalysatoren können als Lösungen in Wasser oder den Fettalkoholen eingesetzt werden, wobei dann der Katalysatorgehalt im Bereich von 10 bis 80 Gew.-% liegen kann. Typische Gehalte für Katalysatoren in Wasser sind 50 bis 80 Gew.-%, in Fettalkoholen 60 bis 90 Gew.-%. Vorzugsweise werden die Katalysatoren in Mengen von 0,1 bis 5, insbesondere 1 bis 3 Gew.-% - bezogen auf die Einsatzstoffe - eingesetzt.

### Acetalisierung

Die Acetalisierung kann diskontinuierlich in einem Rührkessel durchgeführt werden. In einer bevorzugten Ausführungsform der Erfindung erfolgt die Acetalisierung jedoch in einer Kaskade von 3 bis 6, vorzugsweise 3 bis 4 Rührreaktoren. Der Begriff direkte Acetalisierung ist dabei so zu verstehen, daß Glycosesirup und Fettalkohol direkt zu den Zielprodukten umgesetzt werden, also nicht etwa zunächst ein Niedrigalkylglykosid hergestellt wird, welches anschließend mit dem Fettalkohol umacetalisiert werden muß. Die Glucosesirup/Fettalkohol-Suspension wird vor der Acetalisierung entwässert und hierzu beispielsweise der erste Reaktor der Kaskade als Entwässerungsstufe ausgelegt, da ein Wassergehalt in der Mischung zu einem unerwünschten Anstieg des Polyzuckergehaltes im Endprodukt und zu Anbackungen an den Reaktorwänden führen kann. Die Trocknung läßt sich grundsätzlich batchweise als auch kontinuierlich durchführen, dabei sollte bei der batchweisen Durchführung die Temperatur schrittweise erhöht werden, während beim kontinuierlichen Prozeß die Dosierung des Glucosesirup/ Fettalkohol Slurrys vorzugsweise so eingestellt werden sollte, daß der Wassergehalt im Entwässerungsbehälter unterhalb von 0,5 Gew.-% liegt. Da eine gleichmäßig hohe Temperatur in der Reaktionsstufe ebenfalls zu einem Anstieg des Polyzuckergehaltes führen kann, empfiehlt es sich ferner, eine Reaktorkaskade zu verwenden und einen Temperaturgradienten von 70 auf 120°C anzulegen und/oder die Reaktion unter vermindertem Druck durchzuführen. Wahlweise kann man auch einen Druckgradienten von beispielsweise 20 bis 50 mbar anlegen oder unterschiedliche Katalysatorkonzentrationen in den einzelnen Reaktoren einstellen. Nach Abschluß der Acetalisierung empfiehlt es sich, die Reaktionsprodukte durch Zugabe wäßriger Basen wie beispielsweise Natriumhydroxid und/ oder Magnesiumoxid zu neutralisieren, ehe der überschüssige Fettalkohol in an sich bekannter Weise destillativ entfernt wird.

Neben dem Fettalkohol, der als Suspension mit dem Glucosesirup zudosiert wird, läßt sich jedoch falls erforderlich auch noch zusätzlich Fettalkohol hinzugeben.

### Beispiele

**Herstellung der Glucosesirup/Fettalkohol-Suspension.** In einem Rührbehälter wurde Kokosfettalkohol auf 40°C erwärmt und unter starkem Rühren mit Glucosesirup vermischt, wobei die Temperatur unter 45°C gehalten wurde und der Glucosesirup als unterkühlte Schmelze von 30 °C zudosiert wurde. Der Glucosesirup fiel als Feststoff aus und wurde in situ zu einer Glucosesirup/Fettalkohol-Suspension verarbeitet.

**Beispiel 1.** 454 g der Suspension wurde in einem 1-I-Rührreaktor vorgelegt und innerhalb 1h bei 35 mbar auf 75°C aufgeheizt und entwässert. Anschließend wurde die Katalysatorlösung zudosiert und die Mischung auf Reaktionstemperatur erhitzt und bei dieser Temperatur solange gehalten, bis der Restglucosegehalt auf 0,75 Gew.-% abgesunken war.

**Beispiel 2.** 454 g der Suspension wurde in den Entwässerungsbehälter R1 einer Reaktorkaskade bestehend aus 4 1-I-Glasreaktoren eingefüllt und wie in Beispiel 1 beschrieben entwässert. Die übrigen Reaktoren wurden mit Fettalkohol gefüllt. Die Temperierung der Reaktoren erfolgte über Umwälzthermostaten mit Wärmeträgeröl. Als Vakuumpumpe wurde eine Drehschieberölpumpe eingesetzt. Mit einer weiteren Pumpe wurde die Katalysatorlösung in den Reaktor R2 eingebracht. Aus dem Reaktor R4 floß das Reaktionsgemisch in eine Wechselvorlage, in der es mit wäßriger Natriumhydroxidlösung (25 Gew.-%ig) neutralisiert wurde.

Die Versuchsdaten und Ergebnisse der beiden Beispiele sind in Tabelle 1 zusammengefaßt.

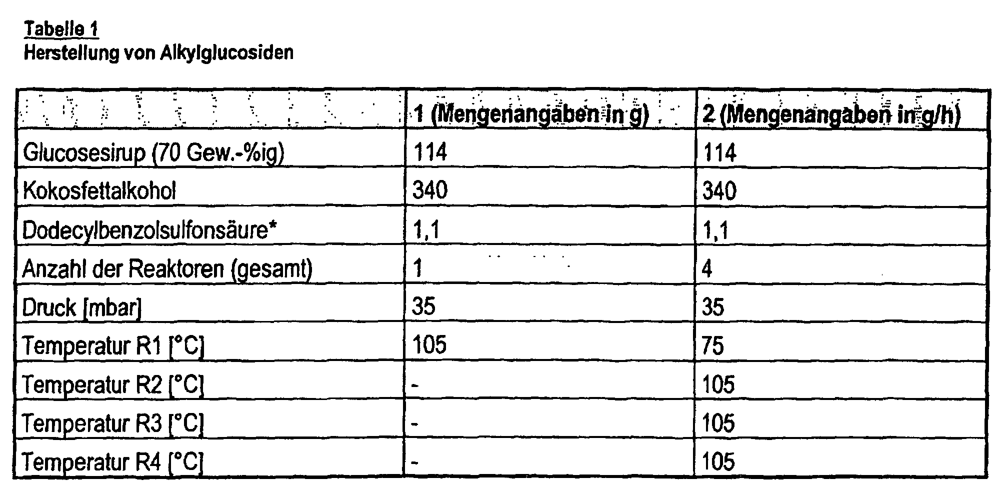

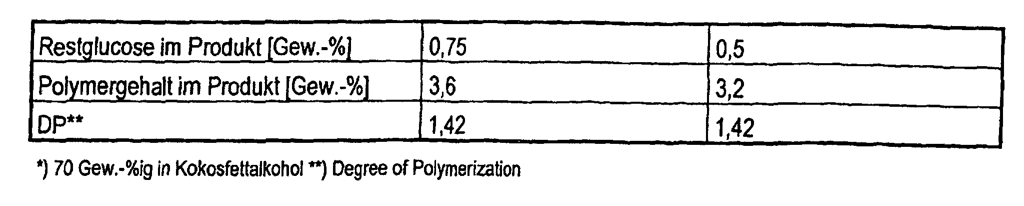

## Patentansprüche

1. Verfahren zur Herstellung von Glucosesuspensionen, bei dem man aufgeschmolzenen Glucosesirup bei 25 bis 45 °C in Fettalkohole einträgt, den als Feststoff ausfallenden Glucosesirup in den Fettalkohol dispergiert und anschließend die Glucose/Fettalkohol-Suspension entwässert.

2. Verfahren nach Anspruch 1, **dadurch gekennzeichnet, dass** man Fettalkohole der Formel (I) einsetzt,
**R**^{**1**}**OH (I)**
in der R¹ für einen aliphatischen, linearen oder verzweigten Kohlenwasserstoffrest mit 6 bis 22 Kohlenstoffatomen und 0 und/oder 1, 2 oder 3 Doppelbindungen steht.

3. Verfahren nach den Ansprüchen 1 und/oder 2, **dadurch gekennzeichnet, dass** man den Glucosesirup und die Fettalkohole im molaren Verhältnis 1 : 1 bis 1 : 10 einsetzt.

4. Verwendung von Suspensionen nach Anspruch 1 als Rohstoffe zur Herstellung von Alkyl- und/oder Alkenyloligoglykosiden.

## Claims

1. A process for the production of glucose suspensions in which melted glucose syrup is introduced into fatty alcohol's at 25 to 45°C, the glucose syrup precipitating as a solid is dispersed in the fatty alcohol and the glucose/fatty alcohol suspension is then freed from water.

2. A process as claimed in claim 1, **characterized in that** fatty alcohol's corresponding to formula **(I)**:
**R**^{**1**}**OH (I)**
in which R¹ is an aliphatic, linear or branched hydrocarbon radical containing 6 to 22 carbon atoms and 0 and/or 1, 2 or 3 double bonds are used.

3. A process as claimed in claims 1 and 2, **characterized in that** the glucose syrup and the fatty alcohol's are used in a molar ratio of 1:1 to 1:10.

4. The use of suspensions produced as claimed in claim 1 as raw materials for the production of alkyl and/or alkenyl oligoglycosides.

## Revendications

1. Procédé de préparation de suspension de glucose dans lequel on introduit un sirop de glucose fondu à 25 à 45°C, dans les alcools gras on disperse dans l'alcool gras le sirop de glucose qui précipite sous forme de matière solide et ensuite on déshydrate la suspension glucose/alcool gras.

2. Procédé de préparation de suspension de glucose selon la revendication 1, **caractérisé en ce qu'** on met en oeuvre des alcools gras de formule I
**R**^{**1**}**OH (I)**
dans laquelle R¹ représente un reste d'hydrocarbure aliphatique, linéaire ou ramifié ayant de 6 à 22 atomes de carbone et 0 et/ou 1, 2, ou 3 double liaisons.

3. Procédé de préparation de suspension de glucose selon la revendication 1 et/ou la revendication 2, **caractérisé en ce qu'** on met en oeuvre le sirop de glucose et les alcools gras dans un rapport molaire de 1 : 1 à 1 : 10.

4. Utilisation de suspensions selon la revendication 1, en tant que matières premières pour la préparation d'alkyl et/ou d'alkényloligoglycosides.
